# EUROPEAN PATENT APPLICATION

(11) **EP 4 785 883 A1**
(43) Date of publication of application: **05.08.2026**
(21) Application number: 23953389.6
(22) Date of filing: 26.09.2023
(51) Int. Cl.: A61B 17/42

(54) **VAGINAL CANNULA AND LAPAROSCOPIC SURGERY SYSTEM**

(71) Applicant: MediConcepts Limited, Hong Kong (HK)
(72) Inventor: CHAN, Wai Kit Benjamin, Hong Kong (HK); FUNG, Ho Wing, Hong Kong (HK); CHAN, Wai Ting, Hong Kong (HK); SZETO, Kwun Kuen, Hong Kong (HK)
(74) Representative: Hoffmann Eitle
(86) International application number: PCT/CN2023/121506
(87) International publication number: WO 2025/065228

(57) **Abstract**

A vaginal cannula (1), comprising a lighting module (10). The lighting module (10) comprises: an incident end (101); a light guide portion (102), one end of which is connected to the incident end (101); and a lighting end (103), which is connected to the other end of the light guide portion (102) and is arranged at the proximal end (120) of the vaginal cannula (1). Light from the incident end (101) is directed to the lighting end (103) via the light guide portion (102), and is emitted from the lighting end (103). A laparoscopic surgery system (100), comprising: a vaginal cannula (1); an endoscope (2); and a robotic arm (3), which is connected to the endoscope (2), wherein the robotic arm (3) is configured to adjust the viewing direction of the endoscope (2); and the robot arm (3) is configured to adjust the viewing direction of the endoscope (2) to be aligned with the area indicated by a visual marker formed by the lighting module (10) of the vaginal cannula (1).

## Description

### TECHNICAL FIELD

The present application relates to the technical field of surgical instruments, and more particularly, to a transvaginal tube and a laparoscopic surgical system.

### BACKGROUND

In laparoscopic hysterectomy, it is necessary to perform an incision to separate the cervix from the vagina. However, it is difficult to quickly and accurately locate the target area for surgery through a laparoscope inserted into an abdominal cavity, which increases the difficulty of the operation and reduces the safety of the operation, for example, an inadvertent uterine perforation or incorrect location of the incision may even occur in extreme cases.

### SUMMARY

In view of the above problems, it is necessary to provide a transvaginal tube and a laparoscopic surgical system. During a surgery using the transvaginal tube, the transvaginal tube with a lighting module is inserted into the patient's vagina, and light from the lighting module travels through the vaginal wall at the cervical-vaginal junction (fornix) to create a location-indicating visual marker. The location-indicating visual marker can be observed through the laparoscope inserted into the abdominal cavity to precisely locate the target area for surgery. Thus, the surgical operator (for example, surgeon or surgical assistant) can easily and quickly locate the target area for surgery, which reduces the surgery difficulty and improves the safety of surgery.

According to one aspect of the present application, a transvaginal tube including the lighting module is provided. The lighting module includes: an incident end; a light guide portion, an end of the light guide portion being connected to the incident end; and an illuminating end connected to the other end of the light guide portion and disposed on a proximal end of the transvaginal tube. Light from the incident end is transmitted to the illuminating end via the light guide portion and is emitted through the illuminating end for illumination.

The transvaginal tube with the lighting module according to an embodiment of the present application is capable of forming a location-indicating visual marker on a patient's vaginal wall during the surgery (for example, laparoscopic hysterectomy). A laparoscope inserted into the abdominal cavity is able to observe the location-indicating visual marker to precisely locate the target area for surgery. Thus, the surgical operator (for example, surgeon or surgical assistant) can easily and quickly locate the target area for surgery, which reduces the surgery difficulty and improves the safety of surgery.

In an embodiment, the lighting module further includes a light emitting portion disposed at the incident end.

In an embodiment, the transvaginal tube further includes a power supply unit electrically connected to the light emitting portion for supplying energy to the light emitting portion.

In an embodiment, the incident end is connected to an external light source.

In an embodiment, the lighting module further includes a fixing portion disposed at the illuminating end and configured to fix the lighting module to an inner wall of the transvaginal tube.

In an embodiment, the incident end is disposed on the distal end of the transvaginal tube, and the light guide portion includes a base and a tube body of the incident end.

In an embodiment, the illuminating end includes a ring-shaped light-emitting member.

In an embodiment, the transvaginal tube further includes: a base provided with a first through hole; and a tube body sleeved with the base and provided with a second through hole. The first through hole and the second through hole are connected to each other and define a third through hole in which the lighting module is arranged.

In an embodiment, the base is provided with a first slot extending along an axial direction of the first through hole, the tube body is provided with a second slot extending along an axial direction of the second through hole, the first slot and the second slot are arranged to be aligned and connected to each other to define a receiving slot in which the lighting module is received.

In an embodiment, the base includes a first portion and a second portion connected to each other, the first through hole is extended through the first portion and the second portion, the second portion has an outer diameter is slightly less than an inner diameter of the second through hole, the second portion is sleeved within the second through hole of the tube body, and the first slot is disposed at least in the second portion.

In an embodiment, the power supply unit is disposed on the first portion, and the light emitting portion is electrically connected to the power supply unit through the first slot.

In an embodiment, the fixing portion is made of an elastic material and includes an arc-shaped structure, and the fixing portion is pressed against an inner wall of the third through hole by a resilient force to fix the lighting module in the third through hole.

In an embodiment, the fixing portion is provided with a connection hole, and the illuminating end of the lighting module is inserted into the connection hole via the first end of the connection hole to connect the lighting module to the fixing portion.

In an embodiment, the second end of the connection hole opposite the first end is provided with a light-passing hole to allow light emitted from the illuminating end to pass through.

According to another aspect of the present application, a laparoscopic surgical system is provided. The laparoscopic surgical system includes: a transvaginal tube; an endoscope; and a robot arm connected to the endoscope and configured to adjust an orientation of a field of view of the endoscope. The robot arm is at least capable of orienting the field of view of the endoscope to align with an area indicated by the location-indicating visual marker formed by the lighting module of the transvaginal tube.

The laparoscopic surgical system according to an embodiment of the present application allows the surgical operator (for example, surgeon or surgical assistant) to easily and quickly locate the target area for surgery by automatically orienting the field of view of the endoscope toward the area indicated by the location-indicating visual marker formed by the lighting module of the transvaginal tube, which reduces the surgery difficulty and improves the safety of surgery.

improves the visual visibility in the endoscope for the vicinity of the target area of the operation by aligning the field of view of the endoscope toward the area illuminated by the lighting module of the transvaginal tube, which reduces the difficulty of the operation, and improves the safety of the operation.

### BRIEF DESCRIPTION OF THE DRAWINGS

The above and other purposes, features, and advantages of the present application will become clearer through a more specific description of the preferred embodiments shown in the attached drawings. The same reference numerals in all the drawings indicate the same parts, and the drawings are not intentionally scaled to the actual size, with the focus on demonstrating the main idea of the present application.

By reading the detailed description of non-limiting embodiments with reference to the following drawings, the other features, purposes, and advantages of this application will become more apparent.
FIG. 1 is a schematic structural view of a transvaginal tube according to an embodiment of the present application;
FIG. 2 is a schematic structural view of the transvaginal tube shown in FIG. 1 at another perspective;
FIG. 3 is a schematic structural view of the transvaginal tube shown in FIG. 1 at another perspective;
FIG. 4 is a schematic structural view of the transvaginal tube shown in FIG. 1 with the lighting module being removed at another perspective;
FIG. 5 is a schematic structural view of the transvaginal tube shown in FIG. 1 with the lighting module being removed;
FIG. 6 is a schematic structural view of a lighting module according to an embodiment of the present application;
FIG. 7 is a schematic structural view of a base of a transvaginal tube according to an embodiment of the present application;
FIG. 8 is a schematic structural view of the base of the transvaginal tube shown in FIG. 7 at another perspective;
FIG. 9 is a schematic structural view of a base and a lighting module according to an embodiment of the present application;
FIG. 10 is a schematic structural view of a transvaginal tube according to an embodiment of the present application;
FIG. 11 is a schematic structural view of a lighting module in the transvaginal tube shown in FIG. 10;
FIG. 12 is a schematic structural view of a fixing portion of a lighting module according to an embodiment of the present application;
FIG. 13 is a schematic structural view of the fixing portion of the lighting module shown in FIG. 12 at another perspective;
FIG. 14 is a partial exploded view of a transvaginal tube according to an embodiment of the present application;
FIG. 15 is a schematic cross-sectional view of the incident end shown in FIG. 14;
FIG. 16 is a partial exploded view of a transvaginal tube according to an embodiment of the present application;
FIG. 17 is a schematic structural view of a laparoscopic surgical system according to an embodiment of the present application; and
FIG. 18 is a schematic view of an area illuminated by the lighting module according to an embodiment of the present application.

### DETAILED DESCRIPTION OF THE EMBODIMENTS

In order to facilitate the understanding of the present application, a more comprehensive description of the present application is provided below with reference to the relevant attached drawings. The preferred embodiment of the present application is shown in the attached drawings. However, the present application can be implemented in many different forms, not limited to the embodiments described herein. On the contrary, the purpose of providing these embodiments is to make the disclosure of the present application more thorough and comprehensive.

It should be noted that when a component is considered to be "connected" to another component, it may be directly connected to and integrated with another component, or it may be connected to another component by an intermediate element. The terms "mounting", "an end", "other end", and similar expressions used in the present application are for illustrative purposes only.

Unless otherwise defined, all technical and scientific terms used in the present application have the same meanings as those commonly understood by those skilled in the art. The terms used in the description of the present application are only for the purpose of describing specific embodiments and are not intended to limit the present application. The term "and/or" used in the present application is intended to include any of the listed items and all combinations of them.

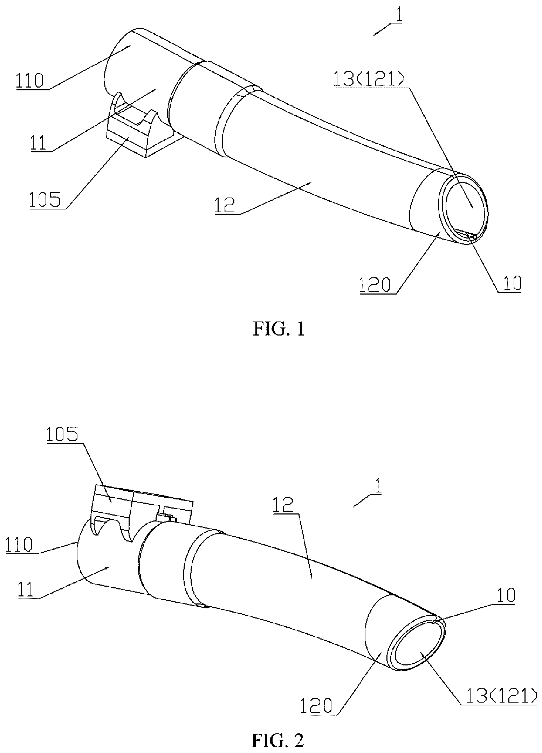

Referring to FIGS. 1 to 3, and 6, an embodiment of the present application provides a transvaginal tube 1 including a lighting module 10, a base 11, and a tube body 12. The base 11 is provided with a first through hole 111. The tube body 12 is sleeved with the base 11, and the tube body 12 is provided with a second through hole 121. The first through hole 111 and the second through hole 121 are connected to each other and define the third through hole 13. The lighting module 10 is arranged in the third through hole 13. The transvaginal tube 1 includes a proximal end 120 and a distal end 110. The proximal end 120 is configured to be inserted into the patient's tissue, such as the vagina. The distal end 110 is an end of the transvaginal tube 1 opposite the proximal end 120.

In an embodiment, the lighting module 10 includes an incident end 101, a light guide portion 102, and an illuminating end 103. An end of the light guide portion 102 is connected to the incident end 101. The illuminating end 103 is connected to the other end of the light guide portion 102 and disposed on the proximal end 120 of the transvaginal tube 1. Light from the incident end 101 is transmitted to the illuminating end 103 via the light guide portion 102 and is emitted from the proximal end 120 of the transvaginal tube 1 through the illuminating end 103. Specifically, when the transvaginal tube 1 is inserted into the patient's tissue, such as the vagina, the light emitted by the illuminating end 103 at the proximal end 120 travels through the wall of human tissue (for example, the vaginal wall), and thus, forming a location-indicating visual marker (for example, a light spot), which provides a visual aid for other surgical instruments inserted into the abdominal cavity. For example, the location-indicating visual marker can be observed through the laparoscope, so that the target area for surgery, such as the location of incision, can be easily and quickly located.

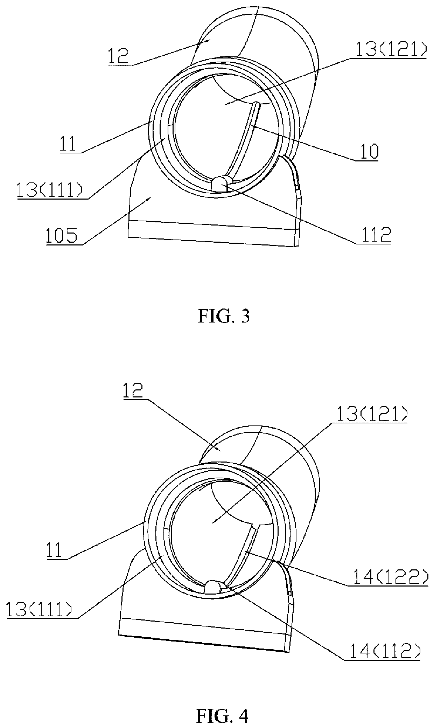

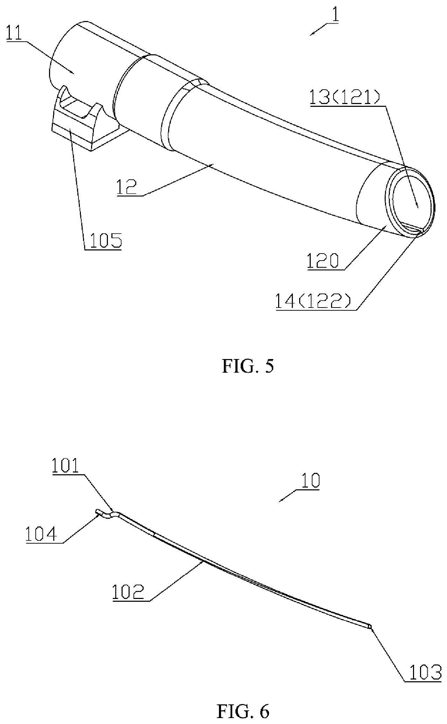

Referring to FIGS. 4 and 5, in an embodiment, the base 11 is provided with a first slot 112 extending in an axial direction of the first through hole 111. The tube body 12 is provided with a second slot 122 extending in an axial direction of the second through hole 121. The first slot 112 and the second slot 122 are arranged to be aligned and connected to each other to define a receiving slot 14 in which the lighting module 10 is received. The receiving slot 14 provides a mounting and fixing space for the lighting module 10, so that the lighting module 10 is capable of entering the patient's body along with the transvaginal tube 1 to form the location-indicating visual marker indicating the target area for surgery on the wall of the human tissue.

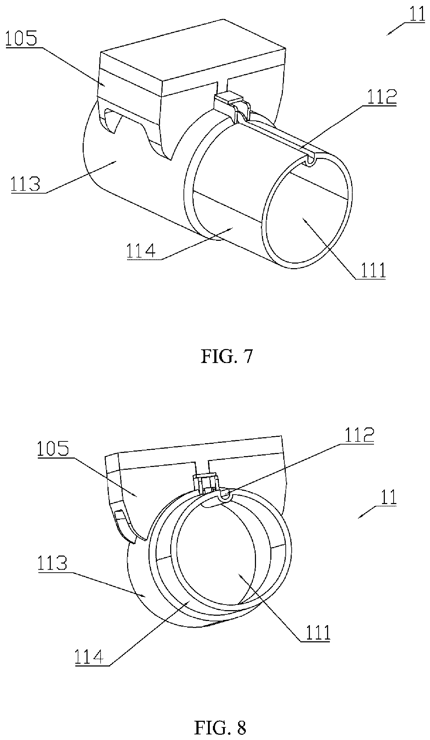

Referring to FIGS. 7 to 9, in an embodiment, the base 11 includes a first portion 113 and a second portion 114 connected to each other. The first through hole 111 is extended through the first portion 113 and the second portion 114. The second portion 114 has an outer diameter slightly less than an inner diameter of the second through hole 121, and the second portion 114 is received within the second through hole 121 of the tube body 12, and the first slot 112 is disposed at least in the second portion 114. The first portion 113 and the second portion 114 form a stepped tubular structure. Specifically, an outer diameter of the first portion 113 is slightly greater than an outer diameter of the second portion 114. This is merely exemplary, and those skilled in the art can rationally set the outer diameter dimensions of the first portion 113 and the second portion 114 as needed.

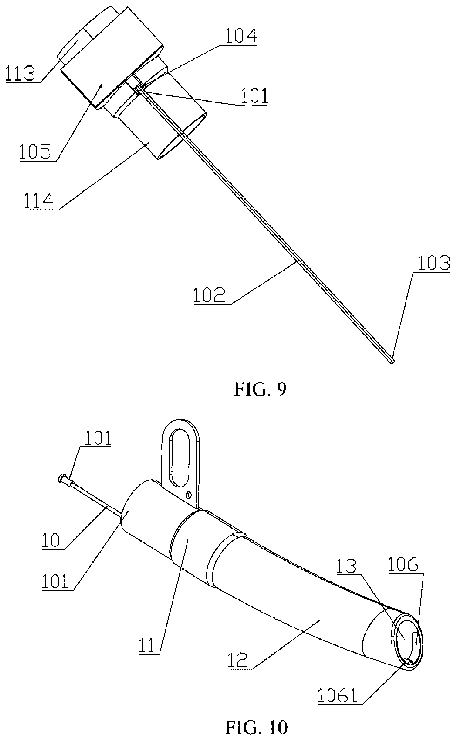

Referring to FIGS. 6 and 9, in an embodiment, the lighting module 10 further includes a light emitting portion 104. The light emitting portion 104 is disposed at the incident end 101. Alternatively, the light emitting portion 104 includes an LED light source. Light emitted by the light emitting portion 104 is guided to the illuminating end 103 via the light guide portion 102, and is emitted through the illuminating end 103 to form the location-indicating visual marker indicating the target area for surgery on the wall of the human tissue.

Referring to FIG. 7, in an embodiment, the lighting module 10 further includes a power supply unit 105 electrically connected to the light emitting section 104 for supplying energy to the light emitting section 104. Alternatively, the power supply unit 105 includes a rechargeable battery. Alternatively, the power supply unit 105 is disposed on an outer surface of the first portion 113, and the light emitting portion 104 is electrically connected to the power supply unit 105 through the first slot 112. It will be appreciated by those skilled in the art that the power supply unit 105 may be disposed in other suitable locations, such as within the first portion 113, as needed.

Referring to FIG. 10, in an embodiment, the incident end 101 of the lighting module 10 may not be provided with the light emitting portion 104, but may also be connected to an external light source. Specifically, the incident end 101 of the lighting module 10 may protrude from the distal end 110 of the transvaginal tube 1 to be connected to the external light source. Light emitted from the external light source is guided to the illuminating end 103 through the light guide portion 102, and is emitted through the illuminating end 103 to form the location-indicating visual marker indicating the target area for surgery on the wall of the human tissue.

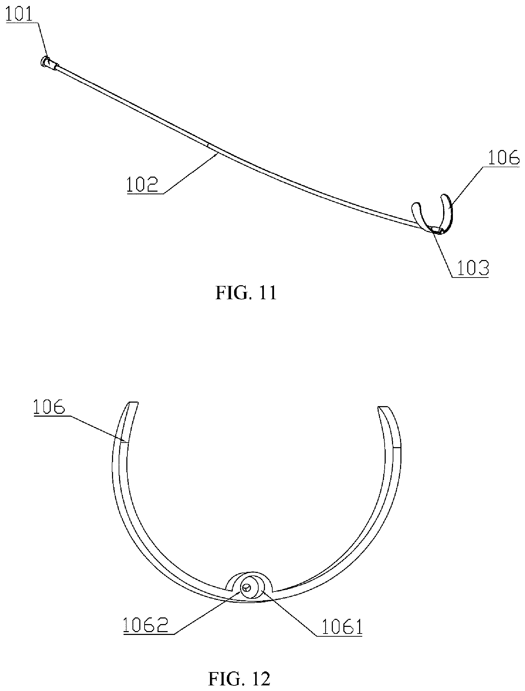

In the embodiment shown in FIGS. 10 and 11, the transvaginal tube 1 is a McCartney Tube^{™}, and the lighting module 10 is fixed to the inner wall of the transvaginal tube 1 by a fixing portion 106 disposed at the illuminating end 103. Specifically, the fixing portion 106 is a generally arc-shaped structure, and is made of an elastic material. A diameter of the arc-shaped structure is slightly greater than that of the third through hole 13. When assembled, the fixing portion 106 is pressed into an end of the transvaginal tube 1 to be entered the patient's body, and the fixing portion 106 is deformed, thereby generating a resilient force. The fixing portion 106 is pressed against the inner wall of the third through hole 13 by the resilient force, thereby fixing the lighting module 10 in the third through hole 13. When detaching, the fixing portion 106 can be detached from the inner wall of the third through hole 13 to remove the lighting module 10 by pressing the arc-shaped structure of the fixing portion 106 toward the center of the third through hole 13.

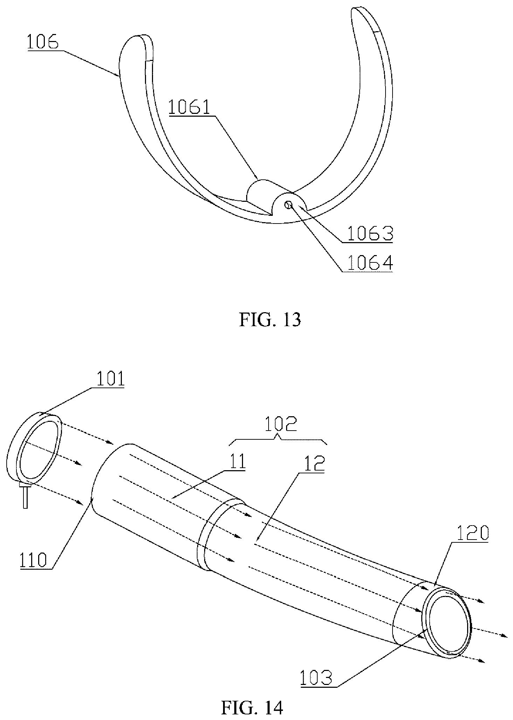

Referring to FIGS. 12 and 13, in an embodiment, the fixing portion 106 is provided with a connection hole 1061, and the illuminating end 103 of the lighting module 10 is inserted into the connection hole 1061 via the first end 1062 of the connection hole 1061, so as to connect the lighting module 10 to the fixing portion 106. The second end 1063 of the connection hole 1061 opposite to the first end 1062 is further provided with a light-passing hole 1064 to allow light emitted from the illuminating end 103 to pass through.

In the embodiment shown in FIGS. 10 to 13, the lighting module 10 is detachably mounted in a transvaginal tube 1, such as a McCartney tube. It is very convenient for the conventional transvaginal tube 1, because it is possible to form the location-indicating visual marker indicating the target area for surgery on the wall of the human tissue, so as to simply and quickly locate the target area for surgery, such as the position of incision, without modifying the structure of the conventional transvaginal tube 1.

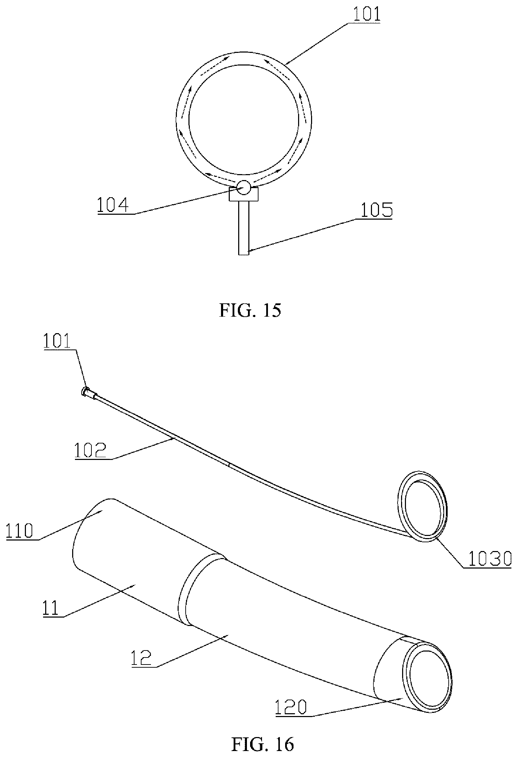

Referring to FIGS. 14 and 15, in an embodiment, the incident end 101 of the lighting module 10 is formed in a ring shape, and is fixed on the distal end 110 of the transvaginal tube 1. At least a part of the base 11 and the tube body 12 of the transvaginal tube 1 are made of a light-guiding material (for example, a transparent material), whereby the base 11 and the tube body 12 are used as the light guide portion 102. In this embodiment, the light emitted by the light emitting portion 104 forms a light evenly distributed in a ring shape through the ring-shaped incident end 101, is transmitted to the illuminating end 103 through the base 11 and the tube body 12, and is emitted from the proximal end 120 of the transvaginal tube 1 through the illuminating end 103. Alternatively, the illuminating end 103 includes a ring-shaped light-emitting member 1030 used to maintain the emitted light as the light evenly distributed in a ring shape. Alternatively, a covering member is disposed on outer surfaces of the base 11 and the tube body 12, so that light is transmitted to the illuminating end 103 only inside the base 11 and the tube body 12. The light emitted by the illuminating end 103 travels through the wall of the human tissue (for example, vaginal wall), and forms the location-indicating visual marker (for example, a light spot) on the wall of the human tissue. The visual location-indicating visual marker provides a visual aid for other surgical instruments inserted into the abdominal cavity. For example, the visual location-indicating visual marker can be observed through the laparoscope, whereby the target area for surgery, such as the location of incision, can be easily and quickly located.

FIG. 16 shows another embodiment of the present application, compared with the embodiments shown in FIGS. 1 to 13, the transvaginal tube 1 in this embodiment has the same structure, the difference lies in that the illuminating end 103 according to this embodiment includes a ring-shaped light-emitting member 1030. The light from the incident end 101 is guided to the ring-shaped light-emitting member 1030 through the light guide portion 102, and is formed into the light evenly distributed in a ring shape through the ring-shaped light-emitting member 1030. The ring-shaped light is suitable in certain application scenarios. For example, if the transvaginal tube 1 does not need to be rotated during the hysterectomy, it is more appropriate to choose the ring-shaped light.

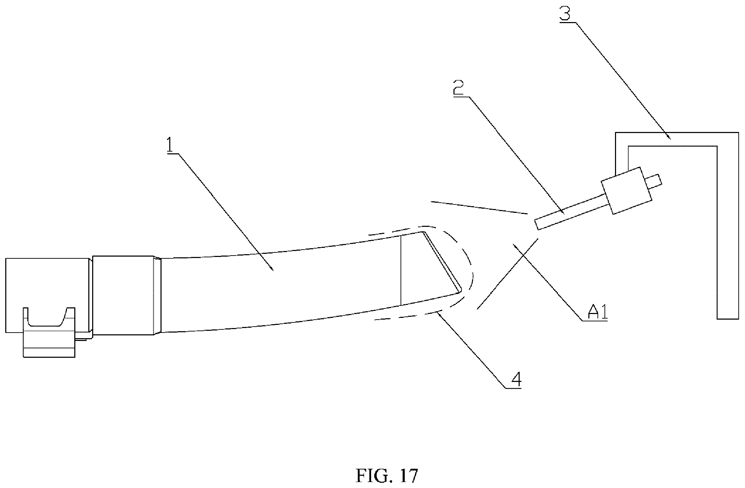

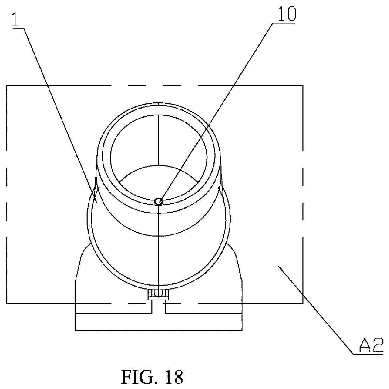

Referring to FIGS. 17 and 18, a laparoscopic surgical system 100 is provided. The laparoscopic surgical system 100 includes a transvaginal tube 1, an endoscope 2, and a robot arm 3. The robot arm 3 is connected to the endoscope 2 and is configured to adjust an orientation of a field of view of the endoscope 2. The robot arm 3 is at least able to adjust the orientation of the field of view of the endoscope 2 to align with an area indicated by the location-indicating visual marker formed by the lighting module 10 of the transvaginal tube 1. In the laparoscopic surgical system 100 provided by an embodiment of the present application, when the transvaginal tube 1 is inserted into the patient's body, for example, into the uterus 4, through operating the robot arm 3, the surgeon may orient the field of view A1 of the endoscope 2 to align with an area A2 indicated by the location-indicating visual marker formed by the lighting module 10 of the transvaginal tube 1 Specifically, during surgery, the surgeon or surgical assistant manipulates the transvaginal tube 1 (e.g., tilts or rotates the transvaginal tube 1 ) to affect the position of the uterus. The endoscope 2 mounted on the robot arm 3 uses the location-indicating visual marker formed by the lighting module 10 as a reference and automatically tracks the transvaginal tube 1, so that the field of view of the endoscope 2 automatically orients the transvaginal tube 1. Therefore, the target area for surgery can be located simply and quickly, the difficulty of surgery is reduced, and the safety of surgery is improved.

The various technical features of the above embodiments can be combined arbitrarily. To make the description concise, all possible combinations of each technical feature in the above embodiments have not been described. However, as long as there is no contradiction in the combination of these technical features, they should be considered within the scope of this description.

The above embodiments only express several embodiments of the present application, and their descriptions are more specific and detailed, but cannot be understood as limiting the patent scope of the present application. It should be pointed out that for Those skilled in the art, several modifications and improvements can be made without departing from the concept of the present application, all of which fall within the protection scope of present application. Therefore, the protection scope of present application should be based on the attached claims.

## Claims

1. A transvaginal tube (1) including a lighting module (10), including:
an incident end (101);
a light guide portion (102), an end of the light guide portion (102) being connected to the incident end (101); and
an illuminating end (103) connected to the other end of the light guide portion (102) and disposed on a proximal end (120) of the transvaginal tube (1),
wherein light from the incident end (101) is transmitted to the illuminating end (103) via the light guide portion (102) and is emitted through the illuminating end (103).

2. The transvaginal tube (1) according to claim 1,
wherein the lighting module (10) further includes a light emitting portion (104) disposed at the incident end (101).

3. The transvaginal tube (1) according to claim 2, further including a power supply unit (105) electrically connected to the light emitting portion (104) for supplying energy to the light emitting portion (104).

4. The transvaginal tube (1) according to claim 1, wherein the incident end (101) is connected to an external light source.

5. The transvaginal tube (1) according to claim 1, further including a fixing portion (106) disposed at the illuminating end (103) and configured to fix the lighting module (10) to an inner wall of the transvaginal tube (1).

6. The transvaginal tube (1) according to any of claims 1 to 5,
wherein the incident end (101) is disposed on a distal end (110) of the transvaginal tube (1), and the light guide portion (102) includes a base (11) and a tube body ( 12) of the transvaginal tube (1).

7. The transvaginal tube (1) according to claim 1,
wherein the illuminating end (103) includes a ring-shaped light-emitting member (1030).

8. The transvaginal tube (1) according to claim 1, further including:
a base (11) provided with a first through hole (111); and
a tube body (12) sleeved with the base (11) and provided with a second through hole (121),
wherein the first through hole (111) and the second through hole (121) are connected to each other and define a third through hole (13) in which the lighting module (10) is arranged.

9. The transvaginal tube (1) according to claim 8, wherein the base (11) is provided with a first slot (112) extending in an axial direction of the first through hole (111), the tube body (12) is provided with a second slot (122) extending in an axial direction of the second through hole (121), the first slot (112) and the second slot (122) are arranged to be aligned and connected to each other to define a receiving slot (14) in which the lighting module (10) is received.

10. The transvaginal tube (1) according to claim 9, wherein the base (11) includes a first portion (113) and a second portion (114) connected to each other, the first through hole (111) is extended through the first portion (113) and the second portion (114), the second portion (114) has an outer diameter slightly less than an inner diameter of the second through hole (121), the second portion (114) is sleeved within the second through hole (121) of the tube body (12), and the first slot (112) is disposed at least in the second portion (114).

11. A transvaginal tube (1) according to claim 10, wherein a power supply unit (105) is disposed on the first portion (113), and the light emitting portion (104) is electrically connected to the power supply unit (105) through the first slot (112).

12. The transvaginal tube (1) according to claim 8, wherein a fixing portion (106) is made of an elastic material and is provided in an arc-shaped structure, and the fixing portion (106) is pressed against an inner wall of the third through hole (13) by a resilient force to fix the lighting module (10) in the third through hole (13).

13. The transvaginal tube (1) according to claim 12, wherein the fixing portion (106) is provided with a connection hole (1061), and the illuminating end (103) of the lighting module (10) is inserted into the connection hole (1061) via a first end (1062) of the connection hole (1061) to connect the lighting module (10) to the fixing portion (106).

14. The transvaginal tube (1) according to claim 13, wherein a second end (1063) of the connecting hole (1061) opposite the first end (1062) is provided with a light-passing hole (1064) to allow the light emitted from the illuminating end (103) to pass through.

15. A laparoscopic surgical system, including:
a transvaginal tube (1) according to any one of claims 1 to 14;
an endoscope (2); and
a robot arm (3) connected to the endoscope (2) and configured to adjust an orientation of a field of view of the endoscope (2),
wherein the robot arm (3) is configured to orient a field of view of the endoscope (2) to align with an area indicated by a location-indicating visual marker formed by the lighting module (10) of the transvaginal tube (1).
